# EUROPÄISCHE PATENTSCHRIFT

(11) **EP 2 664 237 B1**
(45) Veröffentlichungstag und Bekanntmachung des Hinweises auf die Patenterteilung: **02.01.2019**
(21) Anmeldenummer: 13169070.3
(22) Anmeldetag: 11.05.2010
(51) Int. Cl.: A01N 43/08, A01N 31/04, A01N 31/14, A01N 43/16, A61K 8/34, A61K 8/37, A61Q 19/00, A61K 8/49

(54) **Zusammensetzung enthaltend Sorbitanmonocaprylat und Benzylalkohol**
Composition containing sorbitan monocaprylate and benzyl alcohol
Composition contenant du monocaprylate de sorbitane et de l'alcool benzylique

(30) Priorität: 23.05.2009 DE 102009022445
(43) Veröffentlichungstag der Anmeldung: 20.11.2013
(62) Teilanmeldung aus: 10720002.4
(73) Patentinhaber: Clariant International Ltd, 4132 Muttenz (CH)
(72) Erfinder: Klug, Peter, 63762 Grossostheim (DE); Gehm, Sonja, 65812 Bad Soden am Taunus (DE); Kluth, Guiseppina, 65779 Kelkheim (DE); Scherl, Franz-Xaver, 84508 Burgkirchen (DE); Pilz, Maurice Frederic, 60329 Frankfurt am Main (DE)
(74) Vertreter: Paczkowski, Marcus

(56) Entgegenhaltungen:
- EP-A2- 1 813 251
- US-A- 3 331 742
- BACH M ET AL: "KONSERVIERUNGSMITTEL UND IHRE PRAKTISCHE ANWENDUNG IN KOSMETISCHEN PRODUKTEN", SOFW-JOURNAL SEIFEN, OELE, FETTE, WACHSE, VERLAG FUR CHEMISCHE INDUSTRIE, AUGSBURG, DE, Bd. 116, Nr. 9, 13. Juni 1990 (1990-06-13) , Seiten 345-356, XP000134744, ISSN: 0942-7694

## Beschreibung

Die vorliegende Erfindung betrifft flüssige Zusammensetzungen enthaltend Sorbitanmonocaprylat und Benzylalkohol.

Die Verwendung von Alkoholen zum Konservieren kosmetischer, dermatologischer oder pharmazeutischer Formulierungen und Produkte, beispielsweise von Benzylalkohol oder 1,2-Octandiol, ist bekannt (David Steinberg, Preservatives for Cosmetics, Allured Publishing Corporation, zweite Auflage 2006). Die maximale Einsatzkonzentration der Alkohole ist teils durch gesetzliche Regularien, z. B. in der EU durch den ANNEX VI der Kosmetikrichtlinie, vorgegeben. Zum anderen auch durch den viskositätserniedrigenden Effekt der Alkohole in der Formulierung, wenn diese in höheren Konzentrationen eingesetzt werden. Die Erniedrigung der Viskosität ist hierbei ein unerwünschter Nebeneffekt. Zudem kann es weiterhin zu Phasenseparationen innerhalb der kosmetischen, dermatologischen oder pharmazeutischen Formulierung kommen.

Nicht alle antimikrobiell wirksamen Alkohole sind dermatologisch und toxikologisch unbedenklich, weshalb die verwendete Menge an diesen Alkoholen in der kosmetischen, dermatologischen oder pharmazeutischen Formulierung so dosiert sein sollte, dass die Formulierung gerade ausreichend gegen mikrobiellen Befall geschützt ist. Allerdings reicht oftmals die maximal zulässige oder die maximale sich nicht negativ auf die Formulierung auswirkende Einsatzkonzentration der Alkohole nicht aus, um die kosmetische, dermatologische oder pharmazeutische Formulierung hinreichend gegen mikrobiellen Befall zu schützen.

EP-A 1 813 251 lehrt kalt herstellbare, niedrigviskose und langzeitstabile kosmetische Ölin-Wasser-Emulsionen, die Sorbitanester enthalten können. Bach et al. (SOFW-Journal Seifen, Öle, Fette, Wachse, Verlag für Chemische Industrie, Augsburg, Bd. 116, Nr. 9, 1990, Seiten 345-356) lehrt allgemein praktische Anwendungen von Konservierungsmitteln in kosmetischen Anwendungen. In US 3 331 742 werden Verfahren zur Herstellung steriler therapeutischer Zusammensetzungen gelehrt, die unterschiedliche Mono- und Diglyceride enthalten können.

Um die Gesamtmenge an antimikrobiell wirksamen Alkoholen in der kosmetischen, dermatologischen oder pharmazeutischen Formulierung gering zu halten, bestand die Aufgabe, eine dermatologisch und toxikologisch unbedenkliche Substanz zu finden, die in synergistischer Weise die antimikrobielle Wirkung der antimikrobiell wirksamen Alkohole unterstützt.

Überraschend wurde gefunden, dass das bereits in der Kosmetik als Tensid und emulgierendes Agens bekannte und verwendete Sorbitanmonocaprylat genau diese Bedingungen erfüllt.

Gelehrt werden daher flüssige Zusammensetzungen enthaltend
a) von 5 bis 95 Gew.-%, bevorzugt von 10 bis 90 Gew.-%, besonders bevorzugt von 20 bis 80 Gew.-% und insbesondere bevorzugt von 30 bis 70 Gew.-% Sorbitanmonocaprylat und
b) von 5 bis 95 Gew.-%, bevorzugt von 10 bis 90 Gew.-%, besonders bevorzugt von 20 bis 80 Gew.-% und insbesondere bevorzugt von 30 bis 70 Gew.-% an einem oder mehreren Alkoholen der Formel (1)

   R-OH (1)

   worin R ein Rest bestehend aus Kohlenstoff-, Wasserstoff- und gegebenenfalls Sauerstoffatomen mit 5 - 12, vorzugsweise 6 - 11, Kohlenstoffatomen ist, und die Kohlenstoffatome untereinander linear, verzweigt und/oder zyklisch über gesättigte, ungesättigte und/oder aromatische Kohlenstoff-Kohlenstoff-Bindungen verknüpft sein können und die Reste auch Ethereinheiten enthalten können und wobei an die einzelnen Kohlenstoffatome Wasserstoffatome und/oder Hydroxygruppen gebunden sein können.

Erfindungsgemäß ist der Alkohol der Formel (1) in einer solchen flüssigen Zusammensetzung Benzylalkohol. Daher betrifft die vorliegende Erfindung eine flüssige Zusammensetzung enthaltend:
a) 5 bis 95 Gew.-% Sorbitanmonocaprylat und
b) 5 bis 95 Gew.-% Benzylalkohol.

Sorbitanmonocaprylat ist dermatologisch wie toxikologisch auch in sehr hohen Einsatzkonzentrationen unbedenklich und unterstützt in synergistischer Weise die antimikrobielle Wirkung von antimikrobiell wirksamen Alkoholen.
Es wurde weiter gefunden, dass Sorbitanmonocaprylat nicht die Viskosität einer kosmetischen, dermatologischen oder pharmazeutischen Formulierung verringert, sondern sogar im Gegenteil leicht verdickende Eigenschaften besitzt. So können im Vergleich zu den antimikrobiell wirksamen Alkoholen deutlich höhere Mengen an Sorbitanmonocaprylat eingesetzt werden, ohne die Viskosität der kosmetischen, dermatologischen oder pharmazeutischen Formulierung zu erniedrigen oder eine Phasenseparation zu begünstigen.
Die für eine hinreichende Konservierung der kosmetischen, dermatologischen oder pharmazeutischen Formulierung benötigte Einsatzkonzentration von antimikrobiell wirksamen Alkoholen kann in der Kombination mit Sorbitanmonocaprylat signifikant verringert werden. Dadurch reicht oft die Verwendung eines antimikrobiell wirksamen Alkohols zur Konservierung der kosmetischen, dermatologischen oder pharmazeutischen Formulierung aus.
Sorbitanmonocaprylat ist bei Raumtemperatur flüssig und mit den antimikrobiell wirksamen Alkoholen mischbar.
Vorteilhaft an den erfindungsgemäßen flüssigen und daher leicht handhabbaren Zusammensetzungen ist beispielsweise ihre gute Formulierbarkeit.

Offenbart wird, dass der eine oder die mehreren Alkohole der Formel (1) vorzugsweise ausgewählt aus der Gruppe bestehend aus aromatischen Alkoholen, Alkandiolen und Alkantriolen sein können und besonders bevorzugt der eine oder die mehreren Alkohole der Formel (1) ausgewählt aus der Gruppe bestehend aus aromatischen Alkoholen und Alkandiolen sein können.

Es wird offenbart, dass der eine oder die mehreren Alkohole der Formel (1) ausgewählt aus der Gruppe der Alkohole bestehend aus Benzylalkohol, Phenoxyethanol, Propylene Phenoxyethanol, Phenethylalkohol, 1,2-Pentandiol, 1,2-Hexandiol, 1,6-Hexandiol, 1,2-Octandiol, 1,2-Decandiol, Methylpropandiol und Ethylhexylglycerin sein kann/können. Erfindungsgemäß ist der Alkohol der Formel (1) Benzylalkohol .
In einer bevorzugten Ausführungsform der Erfindung enthält Komponente b) der erfindungsgemäßen flüssigen Zusammensetzungen mindestens 50 Gew.-%, besonders bevorzugt 60 bis 100 Gew.-% und insbesondere bevorzugt 100 Gew.-% an einem oder mehreren Alkoholen der Formel (1) ausgewählt aus aromatischen Alkoholen, wobei der eine oder die mehreren aromatischen Alkohole erfindungsgemäß 5 bis 95 Gew.-% Benzylalkohol bezogen auf die gesamte flüssige Zusammensetzung umfasst. Unter den soeben genannten erfindungsgemäßen flüssigen Zusammensetzungen sind in einer bevorzugten Ausführungsform der Erfindung wiederum diejenigen bevorzugt, wobei Komponente b) neben Benzylalkohol einen oder mehrere nichtaromatische Alkohole, vorzugsweise ausgewählt aus der Gruppe der Alkandiole bestehend aus 1,2-Hexandiol, 1,6-Hexandiol, 1,2-Octandiol, 1,2-Decandiol und Ethylhexylglycerin, enthält.
Ferner wird gelehrt, dass die Komponente b) der flüssigen Zusammensetzungen einen oder mehrere Alkohole der Formel (1) ausgewählt aus Alkandiolen und Alkantriolen und vorzugsweise Alkandiolen enthalten kann, vorzugsweise mindestens 50 Gew.-%, besonders bevorzugt 60 bis 100 Gew.-% und insbesondere bevorzugt 100 Gew.-% an einem oder mehreren Alkoholen der Formel (1) ausgewählt aus Alkandiolen und Alkantriolen und vorzugsweise Alkandiolen, wobei das eine oder die mehreren Alkandiole vorzugsweise aus 1,2-Hexandiol, 1,6-Hexandiol, 1,2-Octandiol, 1,2-Decandiol, und Ethylhexylglycerin und besonders bevorzugt aus 1,2-Octandiol und Ethylhexylglycerin ausgewählt sein können.
In einer besonders bevorzugten Ausführungsform der Erfindung enthalten die erfindungsgemäßen flüssigen Zusammensetzungen ein oder mehrere weitere Substanzen ausgewählt aus
d) Wasser,
e) antimikrobiellen Wirkstoffen und
f) Hydrotropen,
wobei die antimikrobiellen Wirkstoffe und Hydrotrope zu den Alkoholen der Formel (1) unterschiedlich sind.

Die antimikrobiellen Wirkstoffe der Komponente e) und die Hydrotrope der Komponente f) sind unterschiedlich zu Sorbitanmonocaprylat.

In einer besonders bevorzugten Ausführungsform der Erfindung enthalten die erfindungsgemäßen flüssigen Zusammensetzungen Wasser. In einer hierunter wiederum bevorzugten Ausführungsform der Erfindung ist das Wasser in einer Menge von 0,1 bis 35 Gew.-%, vorzugsweise von 0,1 bis 20 Gew.-% und besonders bevorzugt von 0,5 bis 10 Gew.-% in den erfindungsgemäßen flüssigen Zusammensetzungen enthalten.

In einer weiteren besonders bevorzugten Ausführungsform der Erfindung enthalten die erfindungsgemäßen flüssigen Zusammensetzungen ein oder mehrere antimikrobielle Wirkstoffe, die zu den Alkoholen der Formel (1) unterschiedlich sind.

Diese antimikrobiellen Wirkstoffe sind vorzugsweise ausgewählt aus Piroctone Olamine (2-Aminoethanol-Salz von 1-Hydroxy-4-methyl-6-(2,4,4-trimethylpentyl)-2(1H)-pyridon, Handelsprodukt ist Octopirox®),
Parabenen, wie beispielsweise Methylparaben, Ethylparaben, Propylparaben, Isopropylparaben, Butylparaben, Isobutylparaben, Natrium Methylparaben, Natrium Ethylparaben, Natrium Propylparaben, Natrium Isobutylparaben, Natrium Isopropylparaben oder Natrium Butylparaben,
organischen Säuren und ihren Salzen, wie beispielsweise Benzoesäure, Sorbinsäure, 3-Acetyl-6-methyl-2[H]-pyran-2,4[3H]-dione (Dehydroacetic acid), p-Methoxybenzoesäure, Ameisensäure, Essigsäure, Propionsäure, Milchsäure, Undecensäure, Salicylsäure, Glykolsäure, 2,4-Hexandiensäure und/oder ihre Salze wie beispielsweise Natriumbenzoat, Kaliumsorbat oder Salicylate wie z. B. Natriumsalicylat,
Imidazolidinyl Harnstoff, Diazolidinyl Harnstoff, Iodopropynyl Butylcarbamat, 2-Bromo-2-Nitropropan-1,3-diol, Cetyltrimethylammoniumchlorid, Cetylpyridiniumchlorid, Benzethoniumchlorid, Diisobutylethoxyethyl-dimethylbenzylammoniumchlorid, Diisobutyl-phenoxy-ethoxy-ethyl-dimethylbenzyl-ammoniumchlorid, N-Alkyl-N,N-dimethyl-benzyl-ammoniumchlorid, -bromid, -saccharinat, Trimethylammoniumchlorid, Natriumaluminiumchlorohydroxylactat, Triethylcitrat, Tricetylmethylammoniumchlorid, 2,4,4'-Trichloro-2'-hydroxydiphenylether (Triclosan), 3,4,4'-Trichlorocarbanilid (Triclocarban), Diaminoalkylamid, beispielsweise L-Lysinhexadecylamid, DMDM Hydantoin, Natrium Hydroxymethylglycinat, 2-Hydroxybiphenyl, Chlorbutanulum, 5-Amino-1,3-bis-(2-ethylhexyl)-5-methyl-hexahydropyrimidin, 2,4-Dichlorbenzylalkohol, N-(4-Chlorphenyl-N'-(3,4-dichlorphenyl)harnstoff, 2,4,4'-Trichlor-2'-hydroxydiphenylether, Poly(hexamethylendiguanid)-hydrochlorid, 1,2-Dibrom-2,4-dicyanobutan, 4,4-Dimethyl-1,3-oxazolidin, Isothiazolinonen, wie beispielsweise Methylisothiazolinon oder Methylchloroisothiazolinon und Methylisothiazolinon im molaren Verhältnis von 3:1, Chloroxylenol, Citratschwermetallsalzen, Silberchlorid, Piroctose, insbesondere Zinksalzen, Pyrithionen und deren Schwermetallsalzen, insbesondere Zinkpyrithion, Zinkphenolsulfat, Farnesol, Bifonazole, Butoconazole, Cloconazole, Clotrimazole, Econazole, Enilconazole, Fenticonazole, Fluconazole, Isoconazole, Itraconazole, Ketoconazol, Miconazole, Naftifine, Oxiconazol, Sulconazole, Terbinafine, Terconazole und Tioconazole und Kombinationen dieser Wirksubstanzen.

In einer insbesondere bevorzugten Ausführungsform der Erfindung enthalten die erfindungsgemäßen flüssigen Zusammensetzungen ein oder mehrere antimikrobielle Wirkstoffe, die zu den Alkoholen der Formel (1) unterschiedlich sind, ausgewählt aus Parabenen, vorzugsweise Methylparaben, Ethylparaben, Propylparaben, Butylparaben, Isobutylparaben, Natrium Methylparaben, Natrium Ethylparaben, Natrium Propylparaben, Natrium Isobutylparaben und/oder Natrium Butylparaben,
organischen Säuren und ihren Salzen, vorzugsweise Benzoesäure, Sorbinsäure, 3-Acetyl-6-methyl-2[H]-pyran-2,4[3H]-dione (Dehydroacetic acid), p-Methoxybenzoesäure, Ameisensäure, Essigsäure, Propionsäure, Milchsäure, Undecensäure, Salicylsäure, Glykolsäure und/oder ihre Salze wie beispielsweise Natriumbenzoat, Kaliumsorbat und/oder Natriumsalicylat,
Formaldehydabspaltern, vorzugsweise Imidazolidinyl Harnstoff, Diazolidinyl Harnstoff, DMDM Hydantoin und/oder Natrium Hydroxymethylglycinat,
halogenierten Konservierungsstoffen, vorzugsweise Iodopropynyl Butylcarbamat und/oder 2-Bromo-2-Nitropropan-1,3-diol und
Isothiazolinonen, vorzugsweise Methylisothiazolinon.

In einer außerordentlich bevorzugten Ausführungsform der Erfindung enthalten die erfindungsgemäßen flüssigen Zusammensetzungen ein oder mehrere antimikrobielle Wirkstoffe, die zu den Alkoholen der Formel (1) unterschiedlich sind, ausgewählt aus organischen Säuren und ihren Salzen, vorzugsweise ausgewählt aus Benzoesäure, Sorbinsäure, 3-Acetyl-6-methyl-2[H]-pyran-2,4[3H]-dione (Dehydroacetic acid), p-Methoxybenzoesäure, Ameisensäure, Essigsäure, Propionsäure, Milchsäure, Undecensäure, Salicylsäure, Glykolsäure und deren Salzen.

Sofern die erfindungsgemäßen flüssigen Zusammensetzungen ein oder mehrere antimikrobielle Wirkstoffe, die zu den Alkoholen der Formel (1) unterschiedlich sind, enthalten, sind diese vorzugsweise in einer Menge von 0,5 bis 30 Gew.-% und besonders bevorzugt von 1,0 bis 25 Gew.-% in den erfindungsgemäßen flüssigen Zusammensetzungen enthalten.

In dem Fall, dass die erfindungsgemäßen flüssigen Zusammensetzungen ein oder mehrere weitere antimikrobielle Wirkstoffe ausgewählt aus Salzen von organischen Säuren enthalten, enthalten die erfindungsgemäßen flüssigen Zusammensetzungen vorzugsweise von 2 bis 35 Gew.-%, besonders bevorzugt von 5 bis 20 Gew.-% und insbesondere bevorzugt von 10 bis 15 Gew.-% Wasser.

In einer weiteren besonders bevorzugten Ausführungsform der Erfindung enthalten die erfindungsgemäßen flüssigen Zusammensetzungen ein oder mehrere Hydrotrope, die zu den Alkoholen der Formel (1) unterschiedlich sind. Diese Hydrotrope sind vorzugsweise ausgewählt aus Xylol-, Toluol- und Cumolsulfonat. Cumolsulfonat ist besonders bevorzugt.

Sofern die erfindungsgemäßen flüssigen Zusammensetzungen ein oder mehrere Hydrotrope, die zu den Alkoholen der Formel (1) unterschiedlich sind, enthalten, ist die Menge des einen oder der mehreren dieser Hydrotrope in den erfindungsgemäßen flüssigen Zusammensetzungen vorzugsweise im Bereich von 1 bis 15 Gew.-%, besonders bevorzugt von 4 bis 10 Gew.-% und insbesondere bevorzugt von 6 bis 8 Gew.-%.

In einer weiteren besonders bevorzugten Ausführungsform der Erfindung enthalten die erfindungsgemäßen flüssigen Zusammensetzungen ein oder mehrere weitere Additive.

Diese weiteren Additive sind vorzugsweise ausgewählt aus Antioxidantien und Solubilisatoren.

In einer weiteren besonders bevorzugten Ausführungsform der Erfindung enthalten die erfindungsgemäßen flüssigen Zusammensetzungen ein oder mehrere Antioxidantien.

Die Antioxidantien sind vorzugsweise ausgewählt aus Superoxid-Dismutase, Tocopherol (Vitamin E), Ascorbinsäure (Vitamin C), Aminosäuren (z. B. Glycin, Histidin, Tyrosin, Tryptophan) und deren Derivaten, Imidazolen (z. B. Urocaninsäure) und deren Derivaten, Peptiden wie D,L-Carnosin, D-Carnosin, L-Carnosin und deren Derivaten (z. B. Anserin), Carotinoiden, Carotinen (z.B. α-Carotin, β-Carotin, Lycopin) und deren Derivaten, Chlorogensäure und deren Derivaten, Liponsäure und deren Derivaten (z. B. Dihydroliponsäure), Aurothioglucose, Propylthiouracil und anderen Thiolen (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salzen, Dilaurylthiodipropionat, Distearylthiodipropionat, Thiodipropionsäure und deren Derivaten (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol/kg), ferner (Metall)-Chelatoren (z. B. α-Hydroxyfettsäuren, Palmitinsäure, Phytinsäure, Lactoferrin), α-Hydroxysäuren (z. B. Citronensäure, Milchsäure, Äpfelsäure), Huminsäure, Phytinsäure, Gallensäure, Gallenextrakten, Bilirubin, Biliverdin, EDTA, EGTA und deren Derivaten, ungesättigten Fettsäuren und deren Derivaten (z. B. γ-Linolensäure, Linolsäure, Ölsäure), Folsäure und deren Derivaten, Ubichinon und Ubichinol und deren Derivaten, Vitamin C und Derivaten (z. B. Ascorbylpalmitat, Mg-Ascorbylphosphat, Ascorbylacetat), Tocopherolen und Derivaten (z. B. Vitamin-E-acetat), Vitamin A und Derivaten (Vitamin-A-palmitat), Koniferylbenzoat des Benzoeharzes, Rutinsäure und deren Derivaten, α-Glycosylrutin, Ferulasäure, Furfurylidenglucitol, Carnosin, Butylhydroxyanisol, Nordihydroguajakharzsäure, Nordihydroguajaretsäure, Trihydroxybutyrophenon, Harnsäure und deren Derivaten, Mannose und deren Derivaten, Zink und dessen Derivaten (z.B. ZnO, ZnSO₄) Selen und dessen Derivaten (z. B. Selenmethionin), Stilbenen und deren Derivaten (z. B. Stilbenoxid, trans-Stilbenoxid) und Superoxid-Dismutase und erfindungsgemäß geeigneten Derivaten (Salze, Ester, Ether, Zucker, Nukleotide, Nukleoside, Peptide und Lipide) dieser genannten Stoffe.

Besonders bevorzugte Antioxidantien sind ausgewählt aus öllöslichen Antioxidantien.

Insbesondere bevorzugte Antioxidantien sind ausgewählt aus Tocopherylacetat und EDTA.

Sofern die erfindungsgemäßen flüssigen Zusammensetzungen ein oder mehrere Antioxidantien enthalten, sind diese vorzugsweise in einer Menge von 0,001 bis 30 Gew.-%, besonders bevorzugt von 0,05 bis 20 Gew.-% und insbesondere bevorzugt von 0,1 bis 5 Gew.-% in den erfindungsgemäßen flüssigen Zusammensetzungen enthalten.

In einer weiteren besonders bevorzugten Ausführungsform der Erfindung enthalten die erfindungsgemäßen flüssigen Zusammensetzungen ein oder mehrere Solubilisatoren.

Bevorzugte Solubilisatoren sind Verbindungen ausgewählt aus der Gruppe bestehend aus Ethanol, Propanol, Isopropanol, n-Butanol, iso-Butanol, Butylenglykol, 1,2-Propylenglykol, Polyethylenglykolen mit einer relativen Molekülmasse von 300 bis 2000, insbesondere mit einer relativen Molekülmasse von 300 bis 600, Triacetin (Glycerintriacetat), 1-Methoxy-2-propanol und PEG-4-Laurat (Polyethylenglykol-4-Laurat).

Besonders bevorzugte Solubilisatoren sind ausgewählt aus Butylenglykol und 1,2-Propylenglykol.

Sofern die erfindungsgemäßen flüssigen Zusammensetzungen ein oder mehrere Solubilisatoren enthalten, sind diese vorzugsweise in einer Menge von 1 bis 20 Gew.-% in den erfindungsgemäßen flüssigen Zusammensetzungen enthalten.

In einer weiteren besonders bevorzugten Ausführungsform der Erfindung enthalten die erfindungsgemäßen flüssigen Zusammensetzungen weniger als 5 Gew.-%, vorzugsweise weniger als 3 Gew.-% und besonders bevorzugt weniger als 1 Gew.-% Wasser. In einer insbesondere bevorzugten Ausführungsform der Erfindung enthalten die erfindungsgemäßen flüssigen Zusammensetzungen kein Wasser, d. h. sie sind wasserfrei.

Vorzugsweise besitzen die erfindungsgemäßen flüssigen Zusammensetzungen ein klares Aussehen.

Die erfindungsgemäßen flüssigen Zusammensetzungen sind in vorteilhafter Weise geeignet zum Konservieren kosmetischer, dermatologischer oder pharmazeutischer Produkte.

Weiterer Gegenstand der Erfindung ist daher die Verwendung einer erfindungsgemäßen flüssigen Zusammensetzung zum Konservieren von kosmetischen, dermatologischen oder pharmazeutischen Produkten, vorzugsweise von Cremes, Cremegelen, Lotionen, Shampoos, Duschbädern, Deodorantien, Antiperspirantien, Feuchttüchern (wet wipes), Sonnenschutzformulierungen oder dekorativen Kosmetikartikeln. In einer bevorzugten Ausführungsform der Erfindung werden kosmetische, dermatologische oder pharmazeutische Formulierungen konserviert.

Die erfindungsgemäßen flüssigen Zusammensetzungen sind des Weiteren in vorteilhafter Weise geeignet zur Herstellung von kosmetischen, dermatologischen oder pharmazeutischen Produkten, vorzugsweise von kosmetischen, dermatologischen oder pharmazeutischen Formulierungen.

Weiterer Gegenstand der vorliegenden Erfindung ist daher die Verwendung einer erfindungsgemäßen flüssigen Zusammensetzung zur Herstellung von kosmetischen, dermatologischen oder pharmazeutischen Produkten und vorzugsweise von kosmetischen, dermatologischen oder pharmazeutischen Formulierungen.

Unter dem Begriff "kosmetische, dermatologische oder pharmazeutische Produkte" werden im Rahmen der vorliegenden Erfindung beispielsweise entsprechende Formulierungen verstanden.

Bei den kosmetischen, dermatologischen oder pharmazeutischen Produkten kann es sich beispielsweise um wässrige, wässrig-alkoholische, wässrig-tensidische oder alkoholische Mittel oder um Mittel auf Ölbasis, inklusive Mittel auf Ölbasis in wasserfreier Form oder um Emulsionen, Suspensionen oder Dispersionen handeln und zwar in Form von Fluids, Schäumen, Sprays, Gelen, Mousse, Lotionen, Cremes, Pudern oder Feuchttüchern (Wet Wipes).

In einer bevorzugten Ausführungsform der Erfindung werden die erfindungsgemäßen flüssigen Zusammensetzungen zum Konservieren von Feuchttüchern verwendet. Hierbei kann es sich bei der auf das textile Gewebe aufgetragenen, zu konservierenden Formulierung um eine Emulsion, insbesondere eine O/W Emulsion, aber auch um eine tensidische Formulierung oder ein öliges Mittel handeln.

In einer weiteren bevorzugten Ausführungsform der Erfindung werden die erfindungsgemäßen flüssigen Zusammensetzungen zum Konservieren von Emulsionen verwendet.

Bei den Emulsionen kann es sich sowohl um Wasser-in-Öl-Emulsionen als auch Öl-in-Wasser-Emulsionen, Mikroemulsionen, Nanoemulsionen und multiple Emulsionen handeln. Die Herstellung der Emulsionen kann in bekannter Weise, d. h. beispielsweise durch Kalt-, Heiß-, Heiß/Kalt- oder PIT-Emulgierung erfolgen. Eine besonders bevorzugte Ausführungsform der Erfindung sind selbstschäumende, schaumförmige, nachschäumende oder schäumbare Emulsionen und Mikroemulsionen.

Gelehrt werden ferner kosmetische, dermatologische oder pharmazeutische Produkte, vorzugsweise kosmetische, dermatologische oder pharmazeutische Formulierungen, die unter Verwendung einer erfindungsgemäßen flüssigen Zusammensetzung hergestellt worden sind, die
a) Sorbitanmonocaprylat und
b) einen oder mehrere Alkohole der Formel (1)

   R-OH (1)

   worin R ein Rest bestehend aus Kohlenstoff-, Wasserstoff- und gegebenenfalls Sauerstoffatomen mit 5 - 12, vorzugsweise 6 - 11, Kohlenstoffatomen ist, und die Kohlenstoffatome untereinander linear, verzweigt und/oder zyklisch über gesättigte, ungesättigte und/oder aromatische Kohlenstoff-Kohlenstoff-Bindungen verknüpft sein können und die Reste auch Ethereinheiten enthalten können und wobei an die einzelnen Kohlenstoffatome Wasserstoffatome und/oder Hydroxygruppen gebunden sein können,
enthält bzw. kosmetische, dermatologische oder pharmazeutische Produkte, vorzugsweise kosmetische, dermatologische oder pharmazeutische Formulierungen, die eine derartige flüssige Zusammensetzung enthalten.

Erfindungsgemäß ist der Alkohol der Formel (1) Benzylalkohol.

Gelehrt werden ferner kosmetische, dermatologische oder pharmazeutische Produkte, vorzugsweise kosmetische, dermatologische oder pharmazeutische Formulierungen, enthaltend
a) Sorbitanmonocaprylat und
b) einen oder mehrere Alkohole der Formel (1)

   R-OH (1)

   worin R ein Rest bestehend aus Kohlenstoff-, Wasserstoff- und gegebenenfalls Sauerstoffatomen mit 5 - 12, vorzugsweise 6 - 11, Kohlenstoffatomen ist, und die Kohlenstoffatome untereinander linear, verzweigt und/oder zyklisch über gesättigte, ungesättigte und/oder aromatische Kohlenstoff-Kohlenstoff-Bindungen verknüpft sein können und die Reste auch Ethereinheiten enthalten können und wobei an die einzelnen Kohlenstoffatome Wasserstoffatome und/oder Hydroxygruppen gebunden sein können.

Erfindungsgemäß ist der Alkohol der Formel (1) Benzylalkohol.

Die kosmetischen, dermatologischen oder pharmazeutischen Produkte, vorzugsweise die kosmetischen, dermatologischen oder pharmazeutischen Formulierungen, können enthalten:
a) Sorbitanmonocaprylat,
b) einen oder mehrere Alkohole der Formel (1)

   R-OH (1)

   worin R ein Rest bestehend aus Kohlenstoff-, Wasserstoff- und gegebenenfalls Sauerstoffatomen mit 5 - 12, vorzugsweise 6 - 11, Kohlenstoffatomen ist, und die Kohlenstoffatome untereinander linear, verzweigt und/oder zyklisch über gesättigte, ungesättigte und/oder aromatische Kohlenstoff-Kohlenstoff-Bindungen verknüpft sein können und die Reste auch Ethereinheiten enthalten können und wobei an die einzelnen Kohlenstoffatome Wasserstoffatome und/oder Hydroxygruppen gebunden sein können, und
e) ein oder mehrere antimikrobielle Wirkstoffe, die zu den Alkoholen der Formel (1) unterschiedlich sind,
wobei der Alkohol der Formel (1) erfindungsgemäß Benzylalkohol ist.

Unter den soeben genannten erfindungsgemäßen kosmetischen, dermatologischen oder pharmazeutischen Produkten, insbesondere den erfindungsgemäßen kosmetischen, dermatologischen oder pharmazeutischen Formulierungen, sind solche bevorzugt, in denen der eine oder die mehreren antimikrobiellen Wirkstoffe der Komponente e) ausgewählt sind aus
Parabenen, vorzugsweise Methylparaben, Ethylparaben, Propylparaben, Butylparaben, Isobutylparaben, Natrium Methylparaben, Natrium Ethylparaben, Natrium Propylparaben, Natrium Isobutylparaben und/oder Natrium Butylparaben,
organischen Säuren und ihren Salzen, vorzugsweise Benzoesäure, Sorbinsäure, 3-Acetyl-6-methyl-2[H]-pyran-2,4[3H]-dione (Dehydroacetic acid), p-Methoxybenzoesäure, Ameisensäure, Essigsäure, Propionsäure, Milchsäure, Undecensäure, Salicylsäure, Glykolsäure und/oder ihre Salze, wie beispielsweise Natriumbenzoat, Kaliumsorbat und/oder Natriumsalicylat,
Formaldehydabspaltern, vorzugsweise Imidazolidinyl Harnstoff, Diazolidinyl Harnstoff, DMDM Hydantoin und/oder Natrium Hydroxymethylglycinat,
halogenierten Konservierungsstoffen, vorzugsweise Iodopropynyl Butylcarbamat und/oder 2-Bromo-2-Nitropropan-1,3-diol und
Isothiazolinonen, vorzugsweise Methylisothiazolinon.

Besonders bevorzugt sind erfindungsgemäße kosmetische, dermatologische oder pharmazeutische Produkte, insbesondere erfindungsgemäße kosmetische, dermatologische oder pharmazeutische Formulierungen, enthaltend
a) Sorbitanmonocaprylat,
b) einen oder mehrere Alkohole der Formel (1)

   R-OH (1)

   worin R ein Rest bestehend aus Kohlenstoff-, Wasserstoff- und gegebenenfalls Sauerstoffatomen mit 5 - 12, vorzugsweise 6 - 11, Kohlenstoffatomen ist, und die Kohlenstoffatome untereinander linear, verzweigt oder zyklisch über gesättigte, ungesättigte oder aromatische Kohlenstoff-Kohlenstoff-Bindungen oder Ethereinheiten verknüpft sind und an die Kohlenstoffatome Wasserstoffatome oder Hydroxygruppen gebunden sind,
   wobei der Alkohol der Formel (1) erfindungsgemäß Benzylalkohol ist, und
e) eine oder mehrere organische Säuren oder deren Salze, vorzugsweise ausgewählt aus Benzoesäure, Sorbinsäure, 3-Acetyl-6-methyl-2[H]-pyran-2,4[3H]-dione (Dehydroacetic acid), p-Methoxybenzoesäure, Ameisensäure, Essigsäure, Propionsäure, Milchsäure, Undecensäure, Salicylsäure, Glykolsäure und deren Salzen.
Bei den Salzen der einen oder mehreren der unter Komponente e) genannten organischen Säuren handelt es sich vorzugsweise
bei Benzoesäure um Natriumbenzoat, Kaliumbenzoat oder Ammoniumbenzoat,
bei Sorbinsäure um Kaliumsorbat oder Ammoniumsorbat,
bei Dehydroacetic acid um Natrium Dehydroacetat, Kalium Dehydroacetat oder Ammonium Dehydroacetat,
bei p-Methoxybenzoesäure um Natrium p-Methoxybenzoat, Kalium p-Methoxybenzoat oder Ammonium p-Methoxybenzoat,
bei Ameisensäure um Natriumformiat, Kaliumformiat oder Ammoniumformiat,
bei Essigsäure um Natriumacetat, Kaliumacetat oder Ammoniumacetat,
bei Propionsäure um Natriumpropionat, Kaliumpropionat, Ammoniumpropionat oder Calciumpropionat,
bei Milchsäure um Natriumlactat, Kaliumlactat, Ammoniumlactat oder Magnesiumlactat, bei Undecensäure um Natriumundecylenat, Kaliumundecylenat, Ammoniumundecylenat, Magnesiumundecylenat oder Zinkundecylenat,
bei Salicylsäure um Natriumsalicylat, Kaliumsalicylat, Ammoniumsalicylat, Magnesiumsalicylat oder Zinksalicylat, und
bei Glykolsäure um Natriumglykolat, Kaliumglykolat, Ammoniumglykolat oder Magnesiumglykolat.

In einer insbesondere bevorzugten Ausführungsform der Erfindung sind die Substanzen der Komponenten a) und b), bezogen auf die fertigen erfindungsgemäßen kosmetischen, dermatologischen oder pharmazeutischen Produkte, vorzugsweise die fertigen erfindungsgemäßen kosmetischen, dermatologischen oder pharmazeutischen Formulierungen, gemeinsam zu 0,1 bis 4,0 Gew.-%, vorzugsweise gemeinsam zu 0,3 bis 3,0 Gew.-%, besonders bevorzugt gemeinsam zu 0,4 bis 2,5 Gew.-% und insbesondere bevorzugt gemeinsam zu 0,5 bis 2,0 Gew.-% in den Produkten bzw. Formulierungen enthalten.

In einer weiteren insbesondere bevorzugten Ausführungsform der Erfindung sind die Substanzen der Komponenten a), b) und e), bezogen auf die fertigen erfindungsgemäßen kosmetischen, dermatologischen oder pharmazeutischen Produkte, vorzugsweise die fertigen erfindungsgemäßen kosmetischen, dermatologischen oder pharmazeutischen Formulierungen, gemeinsam zu 0,1 bis 4,0 Gew.-%, vorzugsweise gemeinsam zu 0,3 bis 3,0 Gew.-%, besonders bevorzugt gemeinsam zu 0,4 bis 2,5 Gew.-% und insbesondere bevorzugt gemeinsam zu 0,5 bis 2,0 Gew.-% in den Produkten bzw. Formulierungen enthalten.

In einer weiteren bevorzugten Ausführungsform der Erfindung handelt es sich bei den erfindungsgemäßen kosmetischen, dermatologischen oder pharmazeutischen Produkten um Rinse-off Produkte, insbesondere um Shampoos, Haarspülungen, Haarkuren, Duschbäder, Duschgels oder Schaumbäder.

In einer weiteren bevorzugten Ausführungsform der Erfindung handelt es sich bei den erfindungsgemäßen kosmetischen, dermatologischen oder pharmazeutischen Produkten um Leave-on Produkte, insbesondere um Tagescremes, Nachtcremes, Pflegecremes, Nährcremes, Bodylotions, Salben oder Lippenpflegemittel. Weitere bevorzugte Leave-on Produkte sind dekorative Kosmetika, insbesondere Make-ups, Eye-shadows, Lippenstifte oder Mascara.

In einer weiteren bevorzugten Ausführungsform der Erfindung handelt es sich bei den erfindungsgemäßen kosmetischen, dermatologischen oder pharmazeutischen Produkten um Sonnenschutzmittel. Diese enthalten einen oder mehrere UV-Filter auf organischer oder anorganischer Basis.

In einer weiteren bevorzugten Ausführungsform der Erfindung handelt es sich bei den erfindungsgemäßen kosmetischen, dermatologischen oder pharmazeutischen Produkten um Deodorantien und Antiperspirantien, insbesondere in Form von Sprays, Sticks, Gelen oder Lotionen.

In einer weiteren bevorzugten Ausführungsform der Erfindung handelt es sich bei den erfindungsgemäßen kosmetischen, dermatologischen oder pharmazeutischen Produkten um tensidfreie Mittel, insbesondere um tensidfreie feste Mittel oder um tensidfreie Emulsionen.

Die erfindungsgemäßen kosmetischen, dermatologischen oder pharmazeutischen Produkte, vorzugsweise die erfindungsgemäßen kosmetischen, dermatologischen oder pharmazeutischen Formulierungen, können als weitere Hilfs- und Zusatzstoffe Tenside, Emulgatoren, kationische Polymere, Verdicker, Filmbildner, antimikrobielle Wirkstoffe, Adstringentien, Antioxidantien, UV-Lichtschutzfilter, Pigmente/Mikropigmente, Gelierungsmittel, sowie weitere in der Kosmetik gebräuchliche Zusätze, wie z. B. Überfettungsmittel, feuchtigkeitsspendende Mittel, Silicone, Stabilisatoren, Konditioniermittel, Glycerin, Konservierungsmittel, Perlglanzmittel, Farbstoffe, Duft- und Parfümöle, Lösungsmittel, Hydrotrope, Trübungsmittel, Fettalkohole, Stoffe mit keratolytischer und keratoplastischer Wirkung, Antischuppenmittel, biogene Wirkstoffe (Lokalanästhetika, Antibiotika, Antiphlogistika, Antiallergica, Corticosteroide, Sebostatika), Vitamine, Bisabolol®, Allantoin®, Phytantriol®, Panthenol®, AHA-Säuren (alpha-Hydroxysäuren), Pflanzenextrakte, beispielsweise Aloe Vera und Proteine enthalten.

In einer bevorzugten Ausführungsform der Erfindung handelt es sich bei den erfindungsgemäßen kosmetischen, dermatologischen oder pharmazeutischen Produkten um entsprechende Formulierungen.

Gelehrt wird ferner die Verwendung von Sorbitanmonocaprylat zur Verbesserung der antimikrobiellen Wirksamkeit von Alkoholen der Formel (1)

R-OH (1)

worin R ein Rest bestehend aus Kohlenstoff-, Wasserstoff- und gegebenenfalls Sauerstoffatomen mit 5 - 12, vorzugsweise 6 - 11, Kohlenstoffatomen ist, und die Kohlenstoffatome untereinander linear, verzweigt und/oder zyklisch über gesättigte, ungesättigte und/oder aromatische Kohlenstoff-Kohlenstoff-Bindungen verknüpft sein können und die Reste auch Ethereinheiten enthalten können und wobei an die einzelnen Kohlenstoffatome Wasserstoffatome und/oder Hydroxygruppen gebunden sein können.

Die Herstellung der erfindungsgemäßen flüssigen Zusammensetzungen kann beispielsweise durch Zusammengeben der einzelnen Komponenten, gegebenenfalls unter leichtem Erwärmen auf ca. 50 °C, erfolgen.

Die nachfolgenden Beispiele und Anwendungen sollen die Erfindung näher erläutern, ohne sie jedoch darauf zu beschränken. Bei allen Prozentangaben handelt es sich um Gewichtsprozent (Gew.-%), sofern nicht explizit anders angegeben.

### Beispiele:

### I) Erfindungsgemäße flüssige Zusammensetzungen

### Beispiele 1 - 12 (Beispiele 1), 6) und 9) sind erfindungsgemäß, die übrigen Beispiele Vergleichsbeispiele)

Zusammensetzungen bestehend aus
1) 50 % Sorbitanmonocaprylat, 50 % Benzylalkohol
2) 50 % Sorbitanmonocaprylat, 50 % Phenoxyethanol
3) 60 % Sorbitanmonocaprylat, 40 % 1,2-Octandiol
4) 40 % Sorbitanmonocaprylat, 30 % Ethylhexylglycerin, 30 % 1,2-Hexandiol
5) 50 % Sorbitanmonocaprylat, 50 % Ethylhexylglycerin
6) 45 % Sorbitanmonocaprylat, 30 % Benzylalkohol, 20 % 1,6-Hexandiol, 5 % Wasser
7) 65 % Sorbitanmonocaprylat, 25 % Phenoxyethanol, 10 % Benzoesäure
8) 70 % Sorbitanmonocaprylat, 30 % 1,2-Decandiol
9) 33 % Sorbitanmonocaprylat, 33 % Benzylalkohol, 34 % 1,2-Octandiol
10) 50 % Sorbintanmonocaprylat, 30 % Phenoxyethanol, 20 % Ethylhexylglycerin
11) 40 % Sorbitanmonocaprylat, 15 % 1,2 Hexandiol, 20 % Ethylhexylglycerin, 20 % Dehydroacetic acid, 5 % Wasser
12) 20 % Sorbitanmonocaprylat, 40 % 1,2-Octandiol, 15 % Phenoxyethanol, 15 % Kaliumsorbat, 10 % Wasser

Die Herstellung der Zusammensetzungen der Beispiele 1 bis 12 erfolgte, indem die einzelnen Komponenten unter Rühren nacheinander am Fingerrührer bei Rührgeschwindigkeiten von 200-300 Umdrehungen/Minute vermengt wurden. Im Falle der Zugabe von organischen Säuren wurde die Zusammensetzung auf etwa 50 °C erwärmt, um eine homogene Mischung zu erhalten.

### II) Synergismus zwischen Sorbitanmonocaprylat und Alkoholen der Formel (1)

Berechnung des Synergistischen Effekts nach der Formel Qₐ/Q_{A} + Q_{b}/Q_{B} = SE (Synergistischer Effekt, nach F.C. Kull et al., Applied Microbiology 1961, 9, 538), wobei Qₐ = Minimale Hemmkonzentration der Verbindung A in % in der eingesetzten Mischung, Q_{A} = Minimale Hemmkonzentration der reinen Substanz A, Q_{b} = Minimale Hemmkonzentration der Verbindung B in % in der eingesetzten Mischung und Q_{B} = Minimale Hemmkonzentration der reinen Substanz B. Bewertung des Synergistischen Effekts: wird ein SE > 1 erhalten, so liegt eine antagonistische Wirkung vor, ist SE = 1, so verhalten sich die Verbindungen gegenüber einander neutral und ist SE < 1 liegt ein synergistischer Effekt vor.

### Beispiel 13

Es wurden Mischungen aus Sorbitanmonocaprylat und Phenoxyethanol oder Benzylalkohol gemäß den Beispielen 1) und 2) untersucht. Die Ergebnisse bezüglich des Synergismus sind in den Tabellen A und B aufgeführt. Mit "MIC Blend" ist dabei die Minimale Hemmkonzentration des jeweiligen Blends bezeichnet.

**Tabelle A Ergebnisse der Untersuchung einer Mischung aus Sorbitanmonocaprylat und Phenoxyethanol im Gewichtsverhältnis 50:50 gemäß Beispiel 2) (Blend) (Vergleichsversuch)**

| | MIC Blend | Sorbitanmonocaprylat | | Phenoxyethanol | | SE |
|---|---|---|---|---|---|---|
| | | Qa | Q_{A} | Q_{b} | Q_{B} | |
| Staphylococcus aureus | 0,063 | 0,0315 | 0,25 | 0,0315 | 0,125 | 0,378 |
| Aspergillus brasiliensis | 0,008 | 0,004 | 0,016 | 0,004 | 0,032 | 0,375 |

**Tabelle B Ergebnisse der Untersuchung einer Mischung aus Sorbitanmonocaprylat und Benzylalkohol im Gewichtsverhältnis 50:50 gemäß Beispiel 1) (Blend)**

| | MIC Blend | Sorbitanmonocaprylat | | Benzylalkohol | | SE |
|---|---|---|---|---|---|---|
| | | Qa | Q_{A} | Q_{b} | Q_{B} | |
| Staphylococcus aureus | 0,032 | 0,016 | 0,25 | 0,016 | 0,25 | 0,128 |
| Aspergillus brasiliensis | 0,008 | 0,004 | 0,016 | 0,004 | 0,063 | 0,313 |

### III) Kosmetische Formulierungen enthaltend flüssige Zusammensetzungen

Von jeder der im Folgenden aufgeführten kosmetischen Formulierungen A - M wurden jeweils 12 verschiedene Formulierungen hergestellt. Und zwar wurde jede kosmetische Formulierung A - M jeweils unter Verwendung der einzelnen flüssigen Zusammensetzungen der Beispiele 1 bis 12 hergestellt.

### Beispiel A - Shampoo

| | | | |
|---|---|---|---|
| A | Genapol® LRO Paste | Clariant | 13,70 % |
| | *Sodium Laureth Sulfate* | | |
| | Genagen® KB | Clariant | 6,00 % |
| | *Coco Betaine* | | |
| | Water | | ad 100 % |
| B | Sodium Chloride | | 1,50 % |
| C | Blend 1-12 | Clariant | 1,50 % |
| D | Citric Acid (10 % in water) | | 0,08 % |

### Herstellung:

- I: Mische die Komponenten von A
- II: Gebe B unter Rühren zu I
- III: Gebe C zu II
- IV: Passe den pH-Wert auf ungefähr 7 an

### Beispiel B-Gesichtsreiniger

| | | | |
|---|---|---|---|
| A | Genapol® LRO liquid | Clariant | 11,10 % |
| | *Sodium Laureth Sulfate* | | |
| | Parfume | | q.s. |
| B | Water | | ad 100 % |
| | Genagen® 3SB | Clariant | 23,30 % |
| | *Coco Betaine, Sodium Cocoyl Isethionate, Sodium Methyl Cocoyl Taurate* | | |
| | Dyestuff solution | | q.s. |
| C | Blend 1-12 | Clariant | 1,20 % |
| D | Citric Acid | | q.s. |

### Herstellung:

- I: Mische die Komponenten A
- II: Gebe die Komponenten von B nach einander in I
- III: Gebe C zu II unter Rühren
- IV: Falls gewünscht, passe den pH-Wert mit C an

### Beispiel C - Aftershave Gel

| | | | |
|---|---|---|---|
| A | Emulsogen® HCU | Clariant | 1,50 % |
| | *Undeceth-8 (and) PEG-40 Hydrogenated Castor Oil* | | |
| B | Tocopherolacetat | | 0,20 % |
| | Menthol | | 0,20 % |
| C | Ethanol | | 30,00 % |
| D | Water | | ad 100 % |
| | Allantoin | Clariant | 0,20 % |
| | *Allantoin* | | |
| | Polyglykol 400 | Clariant | 3,00 % |
| | *PEG-8* | | |
| | Polyglykol 35000 | Clariant | 1,00 % |
| | *PEG-800* | | |
| | Blend 1-12 | Clariant | 2,00 % |
| E | Aristoflex® AVC | Clariant | 1,00 % |
| | *Ammonium Acryloyldimethyltaurate*/*VP Copolymer* | | |

### Herstellung:

- I: Mische A und B und rühre für etwa 5 Minuten
- II: Gebe C zu I und rühre bis die Lösung klar ist
- III: Gebe die Komponenten von D nach einander zu II
- IV: Gebe E zu I und rühre bis eine homogene Formulierung erhalten wird

### Beispiel D - Anti-Ageing Gesichtscreme

| | | | |
|---|---|---|---|
| A | Genapol® T 250 | Clariant | 1,50% |
| | *Cetereth-25* | | |
| | Genapol® DAT | Clariant | 2,00 % |
| | *PEG-150 Polyglyceryl-2 Tristearate and PEG-6* | | |
| | *Caprylic*/*Capric Glyceride* | | |
| B | Water | | ad 100 % |
| C | Aristoflex® AVC | Clariant | 2,00 % |
| | *Ammonium Acryloyldimethyltaurate*/*VP Copolymer* | | |
| D | Glycolic Acid 30 % * | | 6,00 % |
| | Blend 1-12 | Clariant | 1,80 % |

| | | | |
|---|---|---|---|
| * mit NaOH auf pH 4 eingestellt (Gehalt basiert auf freier Glykolsäure) | | | |

### Herstellung:

- I: Löse A in B unter Rühren und leichtem Erwärmen
- II: Gebe C zu I und rühre bis das entstehende Gel frei von Klümpchen ist
- III: Gebe die Komponenten von D zu II und rühre bis die Formulierung homogen ist

### Beispiel E - Emulsion für Baby Wet Wipes

| | | | |
|---|---|---|---|
| A | Propylene Glycol | | 3,00 % |
| | Blend 1-12 | Clariant | 2,00 % |
| | Emulsogen® HCO 040 | Clariant | 1,00 % |
| | *PEG-40 Hydrogenated Castor Oil* | | |
| | Parfume | | 0,20 % |
| B | Hostaphat® KL 340 D | Clariant | 1,50 % |
| | *Trilaureth-4 Phosphate* | | |
| | Velsan® CCT | Clariant | 0,80 % |
| | *Caprylic*/*Capric Triglyceride* | | |
| C | Water | | ad 100 % |
| | Tetrasodium EDTA | | 0,10 % |
| D | Aristoflex® BLV | Clariant | 0,20 % |
| | *Ammonium Acryloyldimethyltaurate*/ *Beheneth- 25 Methacrylate Crosspolymer* | | |
| E | Citric Acid | | q.s |

### Herstellung:

- I: Löse die Komponenten von A
- II: Gebe die Komponenten von B nach einander unter Rühren zu I
- III: Mische die Komponenten von C
- IV: Gebe D zu II
- V: Gebe unter Rühren III zu IV
- VI: Passe den pH-Wert mit E auf etwa pH 6 an

### Beispiel F - O/W Body Lotion

| | | | |
|---|---|---|---|
| A | Velsan® CCT | Clariant | 3,50 % |
| | *Caprylic*/*Capric Triglyceride* | | |
| | Myristyl Myristate | | 2,50 % |
| | Cetearyl Alcohol | | 2,00 % |
| | Glyceryl Stearate Citrate | | 1,00 % |
| | Octyldodecanol | | 1,00 % |
| B | Aristoflex® AVC | Clariant | 0,60 % |
| | *Ammonium Acryloyldimethyltaurate*/*VP Copolymer* | | |
| C | Water | | ad 100 % |
| | Glycerin | | 7,50 % |
| D | Ethanol | | 3,00 % |
| | Dimethicone | | 3,00 % |
| | Tocopheryl Acetate | | 1,00 % |
| | Aloe Barbadensis | | 1,00 % |
| | Blend 1-12 | Clariant | 2,00 % |
| E | Sodium Hydroxide | | q.s. |

### Herstellung:

- I: Schmelze die Komponenten von A bei etwa 70 °C
- II: Mische die Komponenten von C und erhitze die Mischung auf etwa 70 °C
- III: Gebe B zu I wenn I vollständig geschmolzen ist
- IV: Gebe II zu III
- V: Bei 35 °C gebe die Komponenten von D zu IV
- VI: Stelle den pH-Wert mit E auf etwa pH 6,0-6,5 ein

### Beispiel G - Antiperspirant

| | | | |
|---|---|---|---|
| A | Locron® L | Clariant | 30,00 % |
| | *Aluminum Chlorohydrate* | | |
| | Water | | ad 100 % |
| | Polyglykol 400 | Clariant | 3,00 % |
| | *PEG-8* | | |
| | Ethanol | | 17,00 % |
| | Dyestuff solution | | q.s. |
| | Blend 1-12 | Clariant | 1,00 % |
| | Fragrance | | 0,30 % |
| B | Tylose® H 4000 G4 | | 2,50 % |
| | *Hydroxyethlycellulose* | | |

### Herstellung:

- I: Mische die Komponenten von A
- II: Gebe B unter ständigem Rühren zu I. Rühre so lange weiter, bis die Viskosität ihren Endpunkt erreicht hat und die Formulierung homogen ist.

### Beispiel H - Cremespülung

| | | | |
|---|---|---|---|
| A | Genamin® DSAP | Clariant | 2,50 % |
| | *Distearyldimonium Chloride* | | |
| | Genamin® CTAC | Clariant | 3,00 % |
| | *Cetrimonium Chloride* | | |
| | Hostacerin® T- 3 *Ceteareth- 3* | Clariant | 1,50 % |
| | Cetyl Alcohol | | 3,00 % |
| B | Water | | ad 100 % |
| | Blend 1-12 | Clariant | 1,00 % |
| C | Fragrance | | 0,30 % |
| | Dyestuff solution | | q.s. |

### Herstellung:

- I: Schmelze A bei etwa 75 °C
- II: Erhitze B auf etwa 75 °C
- III: Gebe II unter Rühren zu I und rühre bis zum Abkühlen auf 30 °C
- IV: Bei etwa 30 °C gebe C unter Rühren zu II

### Beispiel I - Haarstyling Gel

| | | | |
|---|---|---|---|
| A | Sorbitol | | 5,00 % |
| | Genamin® PQ 43 | Clariant | 0,30 % |
| | Polyquaternium - 43 | | |
| B | Water | | ad 100 % |
| C | Aristoflex® HMB | Clariant | 2,00 % |
| | *Ammonium Acryloyldimethyltaurate*/ *Beheneth- 25* | | |
| | *Methacrylate Crosspolymer* | | |
| D | Aminomethyl Propanol | | 0,30 % |
| | Aristoflex® A 60 | Clariant | 5,00 % |
| | *VA*/*Crotonates Copolymer* | | |
| | Emulsogen® HCO 040 | Clariant | 4,00 % |
| | *PEG-40 Hydrogenated Castor Oil* | | |
| E | Fragrance | | 0,20 % |
| | Blend 1-12 | Clariant | 1,00 % |
| | Dyestuff solution | | q.s. |
| | Timiron Diamond Cluster MP- 149 | | q.s. |
| | *Mica (and) Titanium Dioxide (for EU: CI 77891)* | | |

### Herstellung:

- I: Mische die Komponenten von A
- II: Gebe B zu I
- III: Quelle C in II unter Rühren auf
- IV: Gebe die Komponenten von D eine nach der anderen zu
- V: Gebe die Komponenten von E eine nach der anderen zu IV

### Beispiel J - Make Up Entferner

| | | | |
|---|---|---|---|
| A | Velsan® P8-3 | Clariant | 5,00 % |
| | *Isopropyl C12-15 Pareth-9 Carboxylate* | | |
| B | Hostapon® KCG | Clariant | 2,30 % |
| | *Sodium Cocoyl Glutamate* | | |
| | Genagen® CAB | Clariant | 3,00 % |
| | *Cocamidopropyl Betaine* | | |
| | Genapol® LA 070 | Clariant | 2,00 % |
| | *Laureth-7* | | |
| | Water | | ad 100 % |
| | Allantoin | Clariant | 0,30 % |
| | *Allantoin* | | |
| | Aristoflex® PEA | Clariant | 1,00 % |
| | *Polypropylene Terephthalate* | | |
| | 1,6 Hexanediol | | 2,00 % |
| | 1,2 Propanediol | | 2,00 % |
| | Polyglykol 400 | Clariant | 2,00 % |
| | *PEG-8* | | |
| | Panthenol | | 0,50 % |
| | Lutrol F 127 | | 3,00 % |
| | *Poloxamer 407* | | |
| | Blend 1-12 | Clariant | 1,70 % |

### Herstellung:

- I: Rühre die Komponenten von B nach einander in A und rühre bis eine klare Lösung erhalten wird

### Beispiel K - Lippenglanz

| | | | |
|---|---|---|---|
| A | Versagel® ME 1600 | | ad 100 % |
| | *Hydrogenated polyisobutene (and) Ethylene*/*Propylene*/*Styrene Copolymer (and) Buylene*/*Ethylene*/*Styrene Copolymer* | | |
| | SilCare® Silicone 31M50 | Clariant | 7,00 % |
| | *Caprylyl Trimethicone* | | |
| | SilCare® Silicone 41M65 | Clariant | 3,00 % |
| | *Stearyl Dimethicone* | | |
| | Jojoba Oil | | 2,60 % |
| | Velsan CCT | Clariant | 1,00 % |
| | *Capric*/*Caprylic Triglycerides* | | |
| | Isopropyl Myristate | | 7,40 % |
| B | Gemtone® Tan Opal | | 1,00 to 5,00 % |
| | *Mica and Iron Oxide and TiO₂* | | |
| | Lake - Color | | q.s. |
| C | Blend 1-12 | Clariant | 0,50 % |
| D | Parfum | | q.s. |

### Herstellung:

- I: Erhitze die Komponenten von A auf etwa 80-85 °C und rühre so lange, bis eine homogene Mischung erhalten wird. Lasse diese Mischung auf 70-75 °C abkühlen
- II: Gebe B und C nacheinander unter Rühren zu I und rühre, bis alle Bestandteile gelöst sind
- III: Lasse bis auf 45 °C abkühlen und gebe D zu II, dann fülle die Formulierung in die Gussformen

### Beispiel L - Shimmering Bronze Gel

| | | | |
|---|---|---|---|
| A | Water | | ad 100 % |
| B | Glycerin | | 5,00 % |
| | Polyglykol 35000 S | Clariant | 0,50 % |
| | *PEG-800* | | |
| | Allantoin | Clariant | 0,20 % |
| | *Allantoin* | | |
| C | Aristoflex® AVC | Clariant | 0,60 % |
| | *Ammonium Acryloyldimethyltaurate*/*VP Copolymer* | | |
| | Biron MTU | | 3,00 % |
| | *Bismuth Oxychloride* | | |
| | Flamenco Ultra Silk | | 4,00 % |
| | *Titanium Oxide (and) Mica* | | |
| | Flamenco Sparcle Gold | | 7,00 % |
| | *Mica (and) Iron Oxide (and) Titanium Oxide* | | |
| | Cloisonné Satin Bronze | | 5,00 % |
| | *Iron Oxide (and) Mica* | | |
| | Gemtone Sunstone | | 2,00 % |
| | *Mica (and) Iron Oxide (and) Titanium Oxide* | | |
| | Desert Reflections Canyon Sunset | | 2,00 % |
| | *Mica (and) Iron Oxide (and) Titanium Oxide (and) Tin Oxide* | | |
| | SilCare® Silicone WSI | Clariant | 1,00 % |
| | *proposed INCI: Glyceryl Carboxy Amodimethicone* | | |
| D | Fragrance | | q.s. |
| | Blend 1-12 | Clariant | 1,80 % |

### Herstellung:

- I: Mische die Komponenten von B und löse sie unter Rühren in A
- II: Mische die Komponenten von C und gebe unter leichtem Rühren zu I
- III: Rühre mit höherer Umdrehungszahl (etwa 200 - 250 Umdrehungen/Minute) für etwa zwei Stunden oder bis ein homogenes Gel erhalten wird
- IV: Gebe D unter Rühren zu III

### Beispiel M - Sonnencreme

| | | | |
|---|---|---|---|
| A | SilCare® Silicone WSI | Clariant | 2,00 % |
| | *proposed INCI: Glyceryl Carboxy Amodimethicone* | | |
| | SilCare® Silicone 41M65 | Clariant | 1,00 % |
| | *Stearyl Dimethicone* | | |
| | Dow Corning®246 | | 11,00 % |
| | *Cyclopentasiloxane*/*Cyclohexasiloxane* | | |
| | Titandioxid UV Titan M 262 | | 10,00 % |
| | *Titanium Dioxide*/*Dimethicone* | | |
| | Solaveil CT-100 | | 10,00 % |
| | *C12-15 Alkyl Benzoate*/*Titanium Dioxide*/*Aluminium Stearate*/*Polyhydroxystearic Acid*/*Alumina* | | |
| | Z-Cote HP1 | | 8,00 % |
| | *Zinc Oxide* | | |
| | Butylene Glycol | | 3,00 % |
| | Hostacerin® DGI | Clariant | 3,00 % |
| | *Polyglyceryl-2 Sesquiisostearate* | | |
| | Tegosoft® TN | | 2,00 % |
| | *C12-15 Alkyl Benzoate* | | |
| | Cetiol® 868 | | 2,00 % |
| | *Ethylhexylstearate* | | |
| B | Water | | ad 100 % |
| | Glycerin | | 5,00 % |
| | Ginko Biloba Extract | | 0,70 % |
| | Polyglycose | | 0,20 % |
| | Disodium EDTA | | 0,20 % |
| | Citric Acid | | 0,10 % |
| | Glycerin | | 5,00 % |
| | Ginko Biloba Extract | | 0,70 % |
| C | Tocopheryl Acetate | | 1,00 % |
| | Blend 1-12 | Clariant | 1,80 % |
| | Sodium Chloride | | 1,00 % |
| | Aluminium Hydroxide | | 0,30 % |

### Herstellung:

- I: Schmelze A bei etwa 80 °C
- II: Erwärme B auf etwa 80 °C
- III: Bei einer Rührgeschwindigkeit von etwa 300 Umdrehungen/Minute gebe II zu I. Erhöhe die Rührgeschwindigkeit nach und nach auf 500 Umdrehungen/Minute und behalte bis zum Ende der Formulierungsarbeit diese Geschwindigkeit bei. Lasse die Mischung auf 35 °C abkühlen
- IV: Bei 35 °C gebe C unter Rühren zu III und lasse auf Raumtemperatur abkühlen

Die Zusammensetzungen der Beispiele 1-12 tragen in den kosmetischen Formulierungen A - M zur Erhöhung der Biostabilität bei.

## Patentansprüche

1. Flüssige Zusammensetzung enthaltend:
a) 5 bis 95 Gew.-% Sorbitanmonocaprylat und
b) 5 bis 95 Gew.-% Benzylalkohol.

2. Flüssige Zusammensetzung gemäß Anspruch 1, **dadurch gekennzeichnet, dass** sie eine oder mehrere weitere Substanzen ausgewählt aus:
d) Wasser,
e) antimikrobiellen Wirkstoffen und
f) Hydrotropen enthält,
wobei die antimikrobiellen Wirkstoffe und Hydrotrope zu Alkoholen der folgenden Formel (1) unterschiedlich sind:
R-OH (1)
worin R ein Rest bestehend aus Kohlenstoff-, Wasserstoff- und gegebenenfalls Sauerstoffatomen mit 5-12, vorzugsweise 6-11, Kohlenstoffatomen ist, und die Kohlenstoffatome untereinander linear, verzweigt und/oder zyklisch über gesättigte, ungesättigte und/oder aromatische Kohlenstoff-Kohlenstoff-Bindungen verknüpft sein können und die Reste auch Ethereinheiten enthalten können und wobei an die einzelnen Kohlenstoffatome Wasserstoffatome und/oder Hydroxygruppen gebunden sein können.

3. Flüssige Zusammensetzung gemäß einem der Ansprüche 1 oder 2, **dadurch gekennzeichnet, dass** sie einen oder mehrere antimikrobielle Wirkstoffe, die zu Alkoholen der folgenden Formel (1), wie in Anspruch 2 dargestellt, unterschiedlich sind, in einer Menge von 0,5 bis 30 Gew.-% und bevorzugt von 1,0 bis 25 Gew.-% enthält.

4. Flüssige Zusammensetzung gemäß einem oder mehreren der Ansprüche 1 bis 3, **dadurch gekennzeichnet, dass** sie ein oder mehrere weitere Additive enthält.

5. Flüssige Zusammensetzung gemäß einem oder mehreren der Ansprüche 1 bis 4, **dadurch gekennzeichnet, dass** sie ein klares Aussehen besitzt.

6. Verwendung einer flüssigen Zusammensetzung nach einem oder mehreren der Ansprüche 1 bis 5 zum Konservieren von kosmetischen, dermatologischen oder pharmazeutischen Produkten, vorzugsweise von Cremes, Cremegelen, Lotionen, Shampoos, Duschbädern, Deodorantien, Antiperspirantien, Feuchttüchern (wet wipes), Sonnenschutzformulierungen oder dekorativen Kosmetikartikeln.

7. Verwendung einer flüssigen Zusammensetzung nach einem oder mehreren der Ansprüche 1 bis 5 zur Herstellung von kosmetischen, dermatologischen oder pharmazeutischen Produkten, vorzugsweise von kosmetischen, dermatologischen oder pharmazeutischen Formulierungen.

8. Kosmetische, dermatologische oder pharmazeutische Produkte, enthaltend:
a) Sorbitanmonocaprylat und
b) Benzylalkohol.

9. Kosmetische, dermatologische oder pharmazeutische Formulierungen gemäß Anspruch 8, enthaltend:
a) Sorbitanmonocaprylat und
b) Benzylalkohol.

## Claims

1. Liquid composition containing
a) from 5 to 95% by weight of sorbitan monocaprylate and
b) from 5 to 95% by weight of benzyl alcohol.

2. Liquid composition according to Claim 1, **characterized in that** it contains one or more other substances selected from:
d) water,
e) antimicrobial active ingredients and
f) hydrotropes
in which the antimicrobial active ingredients and hydrotropes are different to the alcohols of the following formula (1):
R-OH (1)
wherein R is a radical consisting of carbon, hydrogen and optionally oxygen atoms having 5 - 12, preferably 6 - 11, carbon atoms, and the carbon atoms can be linked to one another in a linear, branched and/or cyclic manner by means of saturated, unsaturated and/or aromatic carbon-carbon bonds and the radicals can also contain ether units and in which hydrogen atoms and/or hydroxyl groups can be bonded to the individual carbon atoms.

3. Liquid composition according to either of Claims 1 and 2, **characterized in that** it contains one or more antimicrobial active ingredients that are different to the alcohols of the formula (1), as illustrated in Claim 2, in an amount from 0.5 to 30% by weight and preferably from 1.0 to 25% by weight.

4. Liquid composition according to one or more of Claims 1 to 3, **characterized in that** it contains one or more other additives.

5. Liquid composition according to one or more of Claims 1 to 4, **characterized in that** it has a clear appearance.

6. Use of a liquid composition according to one or more of Claims 1 to 5 for preserving cosmetic, dermatological or pharmaceutical products, preferably creams, cream gels, lotions, shampoos, shower baths, deodorants, antiperspirants, wet wipes, sunscreen formulations or decorative cosmetic articles.

7. Use of a liquid composition according to one or more of Claims 1 to 5 for the production of cosmetic, dermatological or pharmaceutical products, preferably of cosmetic, dermatological or pharmaceutical formulations.

8. Cosmetic, dermatological or pharmaceutical products, containing:
a) sorbitan monocaprylate and
b) benzyl alcohol.

9. Cosmetic, dermatological or pharmaceutical formulations according to Claim 8, containing:
a) sorbitan monocaprylate and
b) benzyl alcohol.

## Revendications

1. Composition liquide, contenant :
a) de 5 à 95 % en poids de monocaprylate de sorbitane, et
b) de 5 à 95 % en poids d'alcool benzylique.

2. Composition liquide selon la revendication 1, **caractérisée en ce qu'**elle contient une ou plusieurs substances supplémentaires choisies parmi :
d) de l'eau,
e) les agents actifs microbiens et
f) les hydrotropes,
les agents actifs microbiens et les hydrotropes étant différents des alcools de la formule (1) suivante :
R-OH (1)
dans laquelle R est un radical constitué d'atomes de carbone, d'hydrogène et éventuellement d'oxygène contenant 5 à 12, de préférence 6 à 11, atomes de carbone, et les atomes de carbone peuvent être reliés les uns avec les autres de manière linéaire, ramifiée et/ou cyclique par des liaisons carbone-carbone saturées, insaturées et/ou aromatiques, et les radicaux peuvent également contenir des unités éther, et des atomes d'hydrogène et/ou des groupes hydroxy peuvent être reliés aux atomes de carbone individuels.

3. Composition liquide selon une des revendications 1 ou 2, **caractérisée en ce qu'**elle contient un ou plusieurs agents actifs antimicrobiens, qui sont différents des alcools de formule (1), comme montrée dans la revendication 2, en une quantité de 0,5 à 30 % en poids et de préférence de 1,0 à 25 % en poids.

4. Composition liquide selon une ou plusieurs des revendications 1 à 3, **caractérisée en ce qu'**elle contient un ou plusieurs additifs supplémentaires.

5. Composition liquide selon une ou plusieurs des revendications 1 à 4, **caractérisée en ce qu'**elle présente une apparence limpide.

6. Utilisation d'une composition liquide selon une ou plusieurs des revendications 1 à 5 pour la conservation de produits cosmétiques, dermatologiques ou pharmaceutiques, de préférence de crèmes, de crèmes gels, de lotions, de shampoings, de bains douches, de déodorants, d'antiperspirants, de lingettes humides (wet wipes), de formulations de protection contre le soleil et d'articles cosmétiques décoratifs.

7. Utilisation d'une composition liquide selon une ou plusieurs des revendications 1 à 5 pour la fabrication de produits cosmétiques, dermatologiques ou pharmaceutiques, de préférence de formulations cosmétiques, dermatologiques ou pharmaceutiques.

8. Produits cosmétiques, deratologiques ou pharmaceutiques, contenant :
a) monocaprylate de sorbitane, et
b) l'alcool benzylique.

9. Formulations cosmétiques, deratoligiques ou pharmaceutiques selon la revendication 8, contenant :
a) monocaprylate de sorbitane, et
b) l'alcool benzylique.
